# EUROPEAN PATENT APPLICATION

(11) **EP 4 427 608 A1**
(43) Date of publication of application: **11.09.2024**
(21) Application number: 22888802.0
(22) Date of filing: 12.05.2022
(51) Int. Cl.: A24F 40/40, A24F 40/46, A61M 11/04

(54) **ELECTRONIC ATOMIZATION DEVICE AND ELECTRONIC ATOMIZATION SYSTEM**

(30) Priority: 02.11.2021 CN 202111288738
(71) Applicant: Shenzhen Merit Technology Co., Ltd., Shenzhen, Guangdong 518105 (CN)
(72) Inventor: LIANG, Feng, Shenzhen, Guangdong 518105 (CN); GUO, Conghui, Shenzhen, Guangdong 518105 (CN); LIU, Xiaoli, Shenzhen, Guangdong 518105 (CN); HUANG, Zufu, Shenzhen, Guangdong 518105 (CN)
(74) Representative: Herrero & Asociados, S.L.
(86) International application number: PCT/CN2022/092527
(87) International publication number: WO 2023/077765

(57) **Abstract**

Provided are an electronic atomization device (30) and an electronic atomization system (1), the electronic atomization device comprising a housing (33) and an atomization assembly (10). The housing (33) has an accommodating cavity (36) and an accommodating groove (37); the groove wall of the accommodating groove (37) has a first air inlet (38); the atomization assembly (10) is disposed within the accommodating cavity (36), and is used for atomizing a first aerosol generating substrate so as to generate a first aerosol; an air outlet of the atomization assembly (10) communicates with the first air inlet (38); the accommodating groove (37) is used for accommodating an aerosol generation product (20), and cooperates with the aerosol generation product (20) to form an air guide channel (39), one end of the air guide channel (39) communicates with the first air inlet (38), and the other end thereof extends to a notch of the accommodating groove (37), such that the first aerosol generated by the atomization assembly (10) and a second aerosol generated by the aerosol generation product (20) flow through different air flow channels. Aerosol generated in the atomization assembly (10) may be prevented from entering into the aerosol generation product (20) and being reheated, thereby improving the usage experience of a user.

## Description

### CROSS REFERENCE

The present application claims priority of China Patent Application No. 202111288738.9 filed on November 02, 2021, the entire contents of which are hereby incorporated by reference.

### TECHNICAL FIELD

The present disclosure relates to the technical field of electronic atomizing systems, and in particular to an electronic atomizing device and an electronic atomizing system.

### BACKGROUND

Electronic atomizing systems are configured to atomize aerosol-generating substrates, such as, for roasting a solid substrate of leaves of a plant having a specific fragrance in a heat-not-burning (HNB) manner to form an aerosol, and for heating and atomizing a liquid substrate containing a combination of flavors and fragrances in an electrically heating manner to form an aerosol. Therefore, the electronic atomizing systems can be applied in a variety of different fields.

Currently, with the development of the industry of the electronic atomizing systems, hybrid electronic atomizing systems, combining with the function of roasting the solid substrate of leaves in the HNB manner and the function of heating and atomizing the liquid substrate containing a combination of flavors and fragrances in the electrically heating manner, are the main research direction in the market.

However, existing hybrid electronic atomizing systems are usually a simple combination of an aerosol-generating article containing the solid substrate and an atomizing assembly containing the liquid substrate with flavors and fragrances, so as to generate a mixed aerosol for consumption by a user, with a poor taste that affects the user experience.

### SUMMARY OF THE DISCLOSURE

In view of the above problems, the present disclosure provides an electronic atomizing device and an electronic atomizing system, which may improve the taste of the mixed aerosol, thereby improving the user experience.

To solve the above problems, a technical solution adopted by the present disclosure is to provide an electronic atomizing device, including: a housing, defining a containment cavity and a containment slot; wherein a slot wall of the containment slot defines a first air inlet hole; and an atomizing assembly, arranged in the containment cavity and configured to atomize a first aerosol-generating substrate for generating a first aerosol; wherein the first air inlet hole is configured to be in communication with an air outlet of the atomizing assembly; wherein the containment slot is configured to accommodate an aerosol-generating article and cooperate with the aerosol-generating article to form an air-conducting channel; an end of the air-conducting channel is in communication with the first air inlet hole, and the other end of the air-conducting channel is extended to a slot opening of the containment slot, enabling the first aerosol generated by the atomizing assembly and a second aerosol generated by the aerosol-generating article to flow through different airflow channels.

In some embodiments, the slot wall of the containment slot further defines a second air inlet hole, the second air inlet hole being configured to communicate with an end of the aerosol-generating article near a bottom wall of the containment slot.

In some embodiments, the first air inlet hole and the second air inlet hole are both defined on the bottom wall of the containment slot.

In some embodiments, the atomizing assembly is disposed on the bottom wall of the containment slot, and an outer surface of the bottom wall of the containment slot is arranged with a first air-conducting portion that is in communication with the first air inlet hole; an end of the first air-conducting portion away from the first air inlet hole is connected to the air outlet of the atomizing assembly.

In some embodiments, an inner surface of the bottom wall of the containment slot is arranged with a second air-conducting portion that is in communication with the second air inlet hole; the second air-conducting portion is configured to abut against and be in communication with the end of the aerosol-generating article near the bottom wall of the containment slot, and to positionally limit the aerosol-generating article; the aerosol-generating article is spaced apart from the bottom wall of the containment slot.

In some embodiments, the second air inlet hole includes a plurality of second air inlet holes, and the second air-conducting portion includes a plurality of second air-conducting portions; the plurality of second air inlet holes and the plurality of second air-conducting portions are arranged in one-to-one correspondence; the plurality of second air inlet holes are disposed around the first air inlet hole.

In some embodiments, the housing includes: an outer shell, defining the containment cavity; and an inner tube, disposed at an end of the outer shell and defining the containment slot; wherein an end of the inner tube is inserted into the outer shell, and the other end of the inner tube extends out of the outer shell and forms a nozzle portion.

In some embodiments, the atomizing assembly includes: a liquid storage chamber, configured to store the first aerosol-generating substrate in a liquid state; and an atomizing core, configured to atomize the first aerosol-generating substrate in the liquid state to generate the first aerosol; the electronic atomizing device further includes: a heating assembly, disposed within the housing and configured to heat the aerosol-generating article to generate the second aerosol.

In some embodiments, the heating assembly is arranged on the inner tube, or the inner tube is the heating assembly.

In some embodiments, the electronic atomizing device further includes: a battery; and a control circuit board, electrically connected to the battery, the atomizing core, and the heating assembly; wherein the control circuit board is configured to control the battery to supply power to the heating assembly and the atomizing core, respectively.

To solve the above problems, another technical solution adopted by the present disclosure is to provide an electronic atomizing system, including: the electronic atomizing device as above; and the aerosol-generating article, arranged in the containment slot, spaced apart from a side wall of containment slot, and configured to generate the second aerosol; wherein the electronic atomizing device is configured to heat the aerosol-generating article.

In some embodiments, the aerosol-generating article is detachably arranged in the containment slot and includes: a containment tube and a second aerosol-generating substrate disposed within the containment housing.

In some embodiments, the first aerosol-generating substrate is arranged inside a first end of the containment tube to form a substrate section, a filtering material is arranged inside a second end of the containment tube opposite to the first end to form a filter section, and a hollow section is formed in a middle portion of the containment tube; the substrate section is inserted into the containment slot, and the filter section extends out of the containment slot.

In some embodiments, the aerosol-generating article is spaced apart from a bottom wall of the containment slot, and an end of the aerosol-generating article near the containment slot is a sealed end.

In some embodiments, the aerosol-generating article is spaced apart from a bottom wall of the containment slot; an end of the aerosol-generating article near the bottom wall of the containment slot defines a third air inlet hole; the second air inlet hole and the third air inlet hole are in communication with each other through the second air-conducting portion.

In some embodiments, an end of the aerosol-generating article away from the bottom wall of the containment slot extends out of the nozzle portion.

Compared to the related art, in the present disclosure, each of the electronic atomizing system and the electronic atomizing device includes a housing and an atomizing assembly, the housing defining a containment cavity a containment slot; a slot wall of the containment slot defines a first air inlet hole; the atomizing assembly is arranged in the containment cavity and configured to atomize the first aerosol-generating substrate to generate a first aerosol; an air outlet of the atomizing assembly is in communication with the first air inlet hole; the containment slot is configured to accommodate an aerosol-generating article and cooperate with the aerosol-generating article to form an air-conducting channel; an end of the air-conducting channel is in communication with the first air inlet hole, and the other end of the air-conducting channel is extended to a slot opening of the containment slot, such that the first aerosol generated by the atomizing assembly and a second aerosol generated by the aerosol-generating article flow through different airflow channels. Through the above arrangement, the first aerosol generated by the atomizing assembly and the second aerosol generated by the aerosol-generating article are respectively introduced into the mouth of the user through the slot opening, such that the aerosol generated in the atomizing assembly may be effectively prevented from entering the aerosol-generating article to be heated twice, thereby improving the user experience.

### BRIEF DESCRIPTION OF THE DRAWINGS

FIG. 1 is a structural schematic view of an electronic atomizing system according to some embodiments of the present disclosure.
FIG. 2 is a structural schematic view of an electronic atomizing system according to other embodiments of the present disclosure.
FIG. 3 is a structural schematic view of an atomizing assembly provided according to some embodiments of the present disclosure.
FIG. 4 is a structural schematic view of an aerosol-generating article according to some embodiments of the present disclosure.
FIG. 5 is a structural schematic view of an aerosol-generating article according to other embodiments of the present disclosure.
FIG. 6 is a structural schematic view of an electronic atomizing device according to some embodiments of the present disclosure.
FIG. 7 is a structural schematic view of an aerosol-generating article being inserted into a region A in FIG. 6, according to some embodiments of the present disclosure.
FIG. 8 is a structural schematic view of an aerosol-generating article being inserted into a region A in FIG. 6, according to other embodiments of the present disclosure.
FIG. 9 is an axial sectional view of FIG. 1.
FIG. 10 is an axial sectional view of FIG. 2.
FIG. 11 is a structural schematic view of an aerosol-generating article being inserted into a region A in FIG. 6, according to further other embodiments of the present disclosure.

### DETAILED DESCRIPTION

The technical solutions in the embodiments of the present disclosure will be described clearly and completely in the following in conjunction with the accompanying drawings, and it is obvious that the described embodiments are only a part of the embodiments of the present disclosure, and not all of the embodiments. Based on the embodiments in the present disclosure, all other embodiments obtained by those skilled in the art without making creative labor fall within the scope of the present disclosure.

The terms "first", "second", and "third" in the present disclosure are intended for descriptive purposes only and are not to be construed as indicating or implying relative importance or implicitly specifying the number of technical features indicated. Thus, a feature defined with "first", "second", "third" may expressly or implicitly include at least one of the features. In the description of the present disclosure, "plurality" means at least two, e.g., two, three, etc., unless otherwise expressly and specifically limited. All directional indications (e.g. up, down, left, right, forward, back ......) in the embodiments of the present disclosure are intended only to explain the relative positional relationships, movements, etc. between components in a particular attitude (as shown in the drawings). When the particular attitude changes, the directional indications also change accordingly. The terms "including" and "having" in the embodiments of the present disclosure, and any variations thereof, are intended to cover non-exclusive inclusion. For example, a process, method, system, product, or apparatus including a series of steps or units is not limited to the listed steps or units, but optionally further includes steps or units that are not listed, or optionally includes other steps or components that are inherent to the process, method, product, or apparatus.

Reference to "embodiments" herein implies that particular features, structures, or characteristics described in conjunction with the embodiments may be included in at least one embodiment of the present disclosure. The occurrence of the described phrases at various points in the specification does not necessarily all refer to the same embodiments, nor are they independent or alternative embodiments that are mutually exclusive of other embodiments. It is understood by those skilled in the art, both explicitly and implicitly, that the embodiments described herein may be combined with other embodiments.

The present disclosure is described in detail below in connection with the accompanying drawings and embodiments.

The inventors of the present disclosure have found that the poor taste of existing mixed aerosols is mainly due to the fact that the aerosol formed by the liquid aerosol-generating substrate containing flavors and fragrances is secondarily atomized when it passes through the atomizing assembly containing the solid aerosol-generating substrate. In response thereto, the present disclosure provides a novel electronic atomizing device. The present disclosure is described in detail below in connection with the accompanying drawings and embodiments.

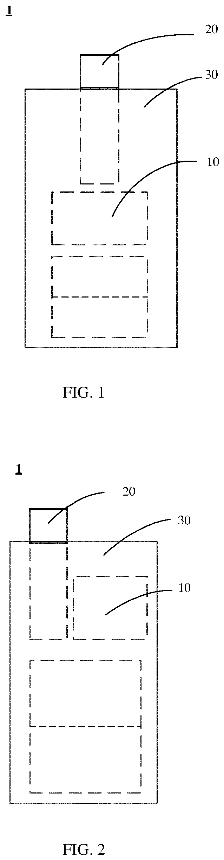

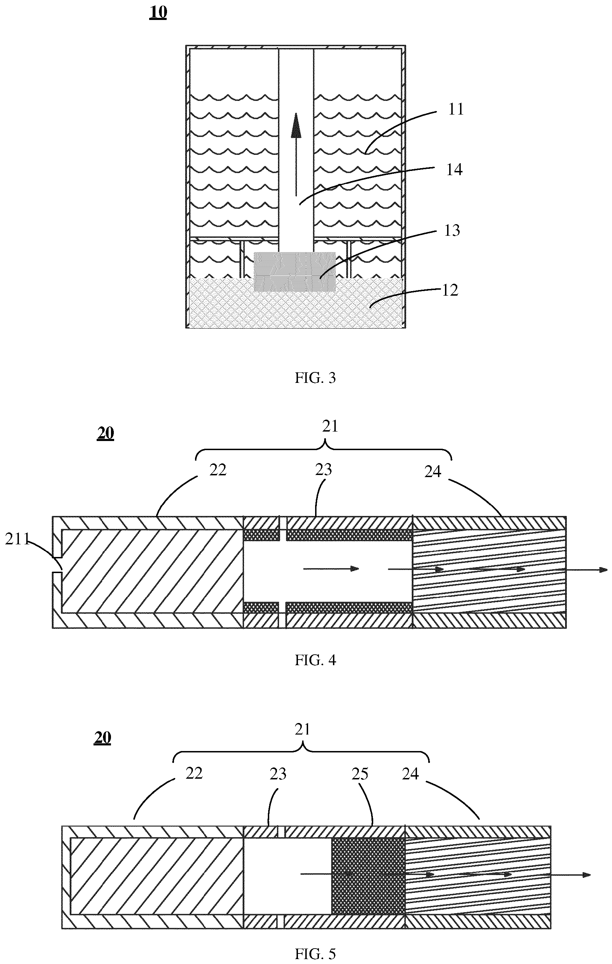

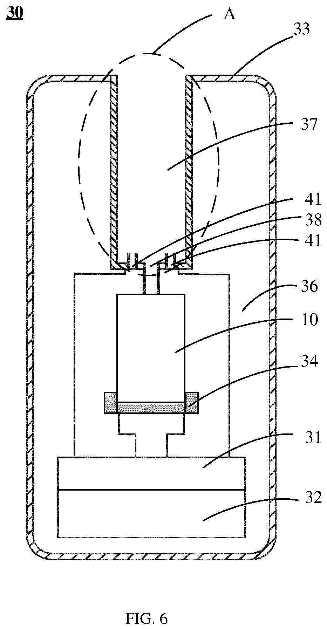

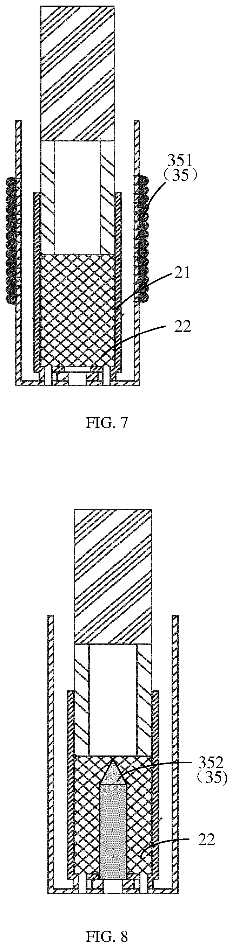

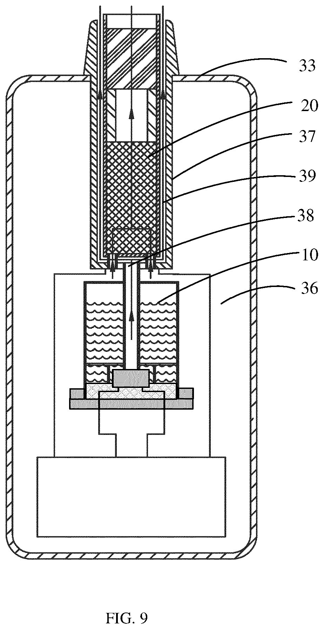

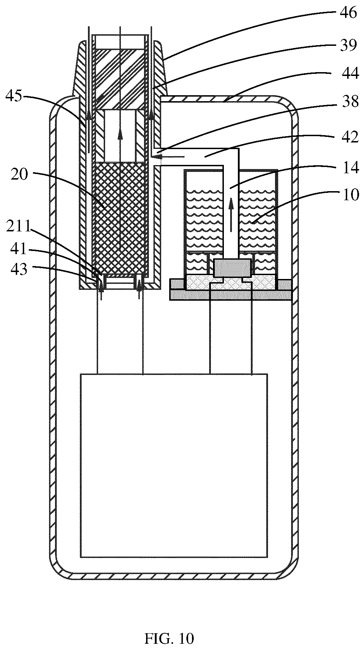

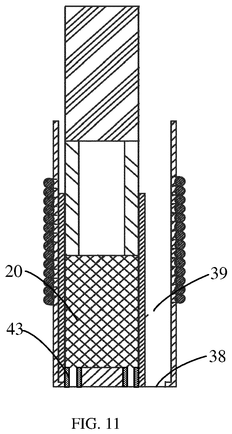

FIG. 1 is a structural schematic view of an electronic atomizing system according to some embodiments of the present disclosure; FIG. 2 is a structural schematic view of an electronic atomizing system according to other embodiments of the present disclosure; FIG. 3 is a structural schematic view of an atomizing assembly provided according to some embodiments of the present disclosure; FIG. 4 is a structural schematic view of an aerosol-generating article according to some embodiments of the present disclosure; FIG. 5 is a structural schematic view of an aerosol-generating article according to other embodiments of the present disclosure; FIG. 6 is a structural schematic view of an electronic atomizing device according to some embodiments of the present disclosure; FIG. 7 is a structural schematic view of an aerosol-generating article being inserted into a region A in FIG. 6, according to some embodiments of the present disclosure; FIG. 8 is a structural schematic view of an aerosol-generating article being inserted into a region A in FIG. 6, according to other embodiments of the present disclosure; FIG. 9 is an axial sectional view of FIG. 1; FIG. 10 is an axial sectional view of FIG. 2; FIG. 11 is a structural schematic view of an aerosol-generating article being inserted into a region A in FIG. 6, according to further other embodiments of the present disclosure.

Referring to FIGS. 1 and 2, an electronic atomizing system 1 is configured to atomize an aerosol-generating substrate, e.g., to roast a solid aerosol-generating substrate in a plant leaf type having a specific fragrance in a heat-not-burning (HNB) manner to cause the solid substrate in the plant leaf type to be roasted to form an aerosol; and/or to heat and atomize a liquid substrate containing a combination of flavors and fragrances to form an aerosol by means of electrical heating or ultrasonic vibration. The electronic atomizing system 1 of the present disclosure can be applied in different fields, such as medical, cosmetic, or recreational vaping. The solid substrate in the plant leaf type having the specific fragrance may generate aerosols with distinct fragrance and high user satisfaction under heating conditions, while the liquid substrate containing the combination of flavors and fragrances has a wide range of aerosol-generating substrates, and thus may generate aerosols with diverse tastes and high atomization volume under heating conditions. The inventors of the present disclosure have combined the advantageous points of both and invented an electronic atomizing system 1.

In some embodiments, the electronic atomizing system 1 includes an aerosol-generating article 20 and an electronic atomizing device 30; the electronic atomizing device 30 includes an atomizing assembly 10, each of the atomizing assembly 10 and the aerosol-generating article 20 accommodating an aerosol-generating substrate; the aerosol-generating substrate can be a solid aerosol-generating substrate in a plant leaf type having a specific fragrance or a liquid aerosol-generating substrate containing a combination of flavors and fragrances; the aerosol-generating substrate can generate an aerosol under a heating condition. The electronic atomizing device 30 is fixedly connected or detachably connected to the aerosol-generating article 20 for providing energy to the aerosol-generating article 20, to heat the aerosol-generating substrate stored within the aerosol-generating article 20. The atomizing assembly 10 in the electronic atomizing device 30 is further configured to heat and atomize the aerosol-generating substrate. The selection is based on the actual situation, for example, when there is a liquid aerosol-generating substrate stored in the atomizing assembly 10 and a solid aerosol-generating substrate stored in the aerosol-generating article 20, the electronic atomizing device 30 is configured to heat the liquid substrate in the atomizing assembly 10 as well as the aerosol-generating article 20 by means of a heating assembly. For another example, the atomizing assembly 10 and the aerosol-generating article 20 are both configured to heat and atomize a liquid aerosol-generating substrate, and the user may choose to store different flavors of the liquid aerosol-generating substrate in the electronic atomizing system 1, and may either vape two flavors of aerosol at the same time or choose one of them according to preference, thereby improving the user's experience.

Referring to FIG. 3, in some embodiments, the atomizing assembly 10 includes a liquid storage chamber 11, an atomizing seat 12, an atomizing core 13, and an atomizing channel 14. The atomizing core 13 may be a porous ceramic core with a screen-printed or coated resistance heating line on a surface of the porous ceramic core, a porous dense substrate core with a screen-printed or coated resistance heating line on a surface of the porous ceramic core, a cotton core externally coiled with a metal resistance wire, or a metal mesh with a set resistance. A first aerosol-generating substrate is stored in the liquid storage chamber 11, where the first aerosol-generating substrate may be a liquid aerosol-generating substrate, and the liquid aerosol-generating substrate may be an oil composed of glycerin, propylene glycol, and flavors and fragrances, etc.; the heating line or metal line, resistance wire, or metal mesh of the atomizing core 13 heats the liquid aerosol-generating substrate under an energized condition to generate a first aerosol, which is exported through the atomizing channel 14.

Referring to FIG. 4, the aerosol-generating article 20 includes a containment tube 21 and a second aerosol-generating substrate disposed within the containment tube 21. The second aerosol-generating substrate may be a solid aerosol-generating substrate. The containment tube 21 may be a cylindrical segmented structure, the solid aerosol-generating substrate (not shown) is arranged inside a first end of the containment tube 21 to form a substrate section 22, a filtering material (not shown) is arranged inside a second end opposite to the first end to form a filter section 24, and a hollow section 23 is formed between the substrate section 22 and the filter section 24.

In some embodiments, the substrate section 22 holds the solid aerosol-generating substrate, and the solid aerosol-generating substrate is not limited to an ordered solid aerosol-generating substrate, a disordered solid aerosol-generating substrate, and a granular solid aerosol-generating substrate. The hollow section 23 may further accommodate a support material (not shown), and the hollow section 23 is configured to collect the aerosol. The filter section 24 accommodates the filtering material, which is configured to filter impurities in the aerosol. The support material and the filter material in the hollow section 23 and the filter section 24 include, but are not limited to, acetate fibers, polylactic acid fibers, polypropylene fibers, paper filter material, and the like. In some embodiments, the first end of the containment tube 21 is of an open-ended structure or defines at least a through hole 211, and outside air can enter the aerosol-generating article 20 through the through hole 211 and carry the second aerosol through the hollow section 23 as well as the filter section 24 into a mouth of the user, thereby lowering the vaping resistance of the aerosol-generating article 20. In other embodiments, the first end of the containment tube 21 is of a sealed structure, such that the user is not affected by an external airflow during vaping, the cracking reaction of the solid aerosol-generated substrate is stable, the vaping consistency is desirable, and the roasting temperature can be further increased under low oxygen, thereby enhancing the fragrance of the solid aerosol-generated substrate. Furthermore, a residue and oily substance of the solid aerosol-generated substrate will not rise with the air to contaminate the device, such that the electronic atomizing device is easy to be cleaned.

Referring to FIG. 5, in some embodiments, in order to prevent the user experience from being affected caused by the temperature of the generated aerosol being too high, a cooling section 25 with a cooling material is further arranged between the hollow section 23 and the filter section 24. The cooling material includes, but is not limited to, polylactic acid, as well as other phase-change materials, etc.

In some embodiments, the liquid aerosol-generating substrate is stored within the atomizing assembly 10, and the solid aerosol-generating substrate is stored within the aerosol-generating article 20. In conjunction with FIG. 6, the electronic atomizing device 30 further includes a control circuit board 31, a battery 32, and a housing 33; where the control circuit board 31 and the battery 32 are accommodated in the housing 33; the control circuit board 31 is configured to control the battery 32 to supply power to the atomizing assembly 10 and to control the battery 32 to heat the aerosol-generating article 20 through the heating assembly. The control circuit board 31 includes multiple control modules that can control an operating temperature of the atomizing assembly 10 and a heating temperature of the aerosol-generating article 20, respectively.

In some embodiments, in conjunction with FIGS. 6 to 8, the electronic atomizing device 30 further includes a connection seat 34 and a heating assembly 35.

The connection seat 34 is arranged in the housing 33 and is electrically connected to the control circuit board 31 and the atomizing assembly 10, for enabling to supply power to the atomizing assembly 10. In a specific embodiment, the connection seat 34 is arranged with a first thimble (not shown), and the first thimble is electrically connected to the battery 32 through the control circuit board 31. The atomizing seat 12 in the atomizing assembly 10 is arranged with planar electrical-connection contacts (not shown), the contacts are electrically connected to the atomizing core 13, and the first thimble is electrically connected to the contacts when the atomizing assembly 10 is assembled into the housing 33, thereby enabling the battery 32 to power and heat the atomizing core 13. The connecting seat 34 may further be arranged with necessary elements such as a microphone, an air-conducting slot, and a magnetic attraction member.

The heating assembly 35 is arranged in the housing 33 and is electrically connected to the control circuit board 31 for heating the aerosol-generating article 20. The heating method of the heating assembly 35 may be electromagnetic heating, resistance heating, or infrared heating, etc. When the heating method of the heating assembly 35 is electromagnetic heating, the heating assembly 35 includes a coil 351 around a circumference of the aerosol-generating article 20, and the containment tube 21 in the aerosol-generating article 20 is a metal material or a metal material is arranged in the substrate section 22 of the aerosol-generating article 20, to generate heat in the electromagnetic field. When the heating method of the heating assembly 35 is infrared heating, the heating assembly 35 includes a tubular heating element (not shown) around the circumference of the aerosol-generating article 20 and an infrared material coated on an outer surface of the heating element. When the heating method of the heating assembly 35 is resistive heating, the heating assembly 35 includes a center needle-like or center sheet-like heating body 352, and the heating body 352 is inserted into the substrate section 22 of the aerosol-generating article 20. The heating method and shape of the heating assembly 35 may be selected according to actual needs and will not be discussed herein.

Referring to FIGS. 3, 6, 9, and 10, in some embodiments, the housing 33 has a containment cavity 36 and a containment slot 37. A slot wall of the containment slot 37 defines a first air inlet hole 38. The containment cavity 36 is configured to accommodate the atomizing assembly 10, and the first air inlet hole 38 is configured to communicate with an air outlet of the atomizing channel 14 of the atomizing assembly 10. The containment slot 37 is configured to accommodate the aerosol-generating article 20 and configured to cooperate with the aerosol-generating article 20 to form an air-conducting channel 39, an end of the air-conducting channel 39 being in communication with the first air inlet hole 38, and the other end of the air-conducting channel 39 extending into a slot opening of the containment slot 37, such that the first aerosol generated by the atomizing assembly 10 and the second aerosol generated by the aerosol-generating article 20 are caused to be supplied to the user through separate airflow channels. Specifically, a side wall and a bottom wall of the aerosol-generating article 20 are spaced apart from a slot wall of the containment slot 37 to cooperate in forming the air-conducting channel 39, and the air-conducting channel 39 and the atomizing channel 14 inside the atomizing assembly 10 are in communication with each other through the first air inlet hole 38 and cooperate in forming a first airflow channel; a second airflow channel is formed inside the aerosol-generating article 20; and the first and the second airflow channels provide the user with aerosols from the atomizing assembly 10 and the aerosol-generating article 20, respectively. Through the above arrangement, the first aerosol generated by the atomizing assembly 10 and the second aerosol generated by the aerosol-generating article 20 are exported through separate airflow channels. Compared to a traditional simple series connection of the atomizing assembly 10 and the aerosol-generating article 20, the electronic atomizing system 1 of the present disclosure may avoid a poor taste of the first aerosol caused by the first aerosol entering the entering the substrate section 22 in the aerosol-generating article 20 and being heated twice; further, the electronic atomizing system 1 may avoid a loss of atomized amount caused by the first aerosol adhering to the solid aerosol-generating substrate within the substrate section 22 in the aerosol-generating article 20. Further, the electronic atomizing system 1 containing both the first aerosol and the second aerosol may maximize the fragrance and atomized amount of the aerosol-generating substrate, thereby increasing user satisfaction.

In some embodiments, the through hole 211 in the aerosol-generating article 20 is in communication with the air-conducting channel 39, such that a portion of the first aerosol generated by the atomizing assembly 10 enters the aerosol-generating article 20, mixes with the second aerosol generated by the aerosol-generating article 20, and then is supplied to the user through the second airflow channel. The other part of the first aerosol generated by the atomizing assembly 10 that does not enter into the aerosol-generating article 20 is supplied to the user through the first airflow channel.

In some embodiments, the slot wall of the containment slot 37 further defines a second air inlet hole 41, the second air inlet hole 41 being configured to communicate with an end of the aerosol-generating article 20 near a bottom wall of the containment slot 37. The second air inlet hole 41 is configured to provide air to the aerosol-generating article 20. Specifically, the second air inlet hole 41 may be defined on the side wall or bottom wall of the containment slot 37.

In some embodiments, in order to reduce the difficulty of processing, as well as to reduce the flow path of the first aerosol and the air entering the aerosol-generating article 20, the first air inlet hole 38 and the second air inlet hole 41 are both defined on the bottom wall of the containment slot 37. In the embodiments, the atomizing assembly 10 is disposed at the bottom of the containment slot 37, and an outer surface of the bottom wall of the containment slot 37 is arranged with a first air-conducting portion 42 that is in communication with the first air inlet hole 38. An end of the first air-conducting portion 42 away from the first air inlet hole 38 is connected to the air outlet of the atomizing assembly 10. The first aerosol generated in the atomizing assembly 10 flows into the mouth of the user through the atomizing channel 14, the first air-conducting portion 42, and the air-conducting channel 39. The first air-conducting portion 42 may be an air-conducting tube or a hollow protrusion for easy insertion into the air outlet of the atomizing channel 14 to ensure a good sealing of the channel. In some embodiments, the aerosol-generating article 20 and the containment slot 37 are cylindrical and coaxial, and the annular air-conducting channel 39 is formed between the side wall of the aerosol-generating article 20 and the side wall of the containment slot 37. The first aerosol generated in the atomizing assembly 10 enters a gap between the bottom wall of the aerosol-generating article 20 and the bottom wall of the containment slot 37 through the internal atomizing channel 14 and the first air-conducting portion 42, uniformly diffuses to the annular air-conducting channel 39, and flows through the annular air-conducting channel 39 into the mouth of the user. Since the annular air-conducting channel 39 is arranged around the aerosol-generating article 20, the mixing uniformity between the first aerosol and the second aerosol generated by the aerosol-generating article 20 in the user's mouth may be improved, thereby improving the user's vaping experience.

In some embodiments, an inner surface of the bottom wall of the containment slot 37 is arranged with a second air-conducting portion 43 in communication with the second air inlet hole 41. The aerosol-generating article 20 is spaced apart from the bottom wall of the containment slot 37, and the through hole 211 on the end of the aerosol-generating article 20 near the bottom wall of the containment slot 37 serves as a third air inlet hole. The second air inlet hole 41 and the through hole 211 are in communication with each other through the second air-conducting portion 43. The second air-conducting portion 43 may be an air-conducting tube or a hollow protrusion.

Specifically, the second air-conducting portion 43 is configured to abut against and be in communication with the end of the aerosol-generating article 20 near the bottom wall of the containment slot 37, and to positionally limit the aerosol-generating article 20, such that the aerosol-generating article 20 is spaced apart from the bottom wall of the containment slot 37. In the embodiments, the side wall of the aerosol-generating article 20 is also spaced apart from the containment slot 37, and the side wall and bottom wall of the aerosol-generating article 20 cooperate with the slot wall of the containment slot 37 to form the air-conducting channel 39. In other embodiments, referring to FIG. 11, the bottom wall of the aerosol-generating article 20 is spaced apart from the bottom wall of the containment slot 37 through the second air-conducting portion 43, and a portion of the aerosol-generating article 20 is spaced apart from the bottom wall of the containment slot 37; a part of the side wall of the aerosol-generating article 20 is attached to the side wall of the containment slot 37, and a remaining part of the side wall of the aerosol-generating article 20 is spaced apart from the containment slot 37. The remaining part of the side wall of the aerosol-generating article 20 cooperates with the slot wall of the containment slot 37 to form the air-conducting channel 39, i.e., the aerosol-generating article 20 is biased within the containment slot 37. In other embodiments, the second air-conducting portion 43 is adopted with a metal conduit, and each of two the second air-conducting portions 43 is electrically connected to the control circuit board 31 to serve as a connecting electrode. A heating film is arranged on an outer surface of the substrate section 22 of the aerosol-generating article 20. When the aerosol-generating article 20 is in contact and in communication with the second air-conducting portion 43, the second air-conducting portion 43 is electrically connected to the heating film on the outer surface of the substrate section 22. In some embodiments, each of the second air inlet hole 41 and the second air-conducting portion 43 is set in plural, and the second air inlet holes 41 and the second air-conducting portions 43 are arranged in one-to-one correspondence. The second air inlet holes 41 are disposed around the first air inlet hole 38, so as to increase the airflow amount and the airflow rate of the outside air into the aerosol-generating article 20, thereby further reducing the vaping resistance of the aerosol-generating article 20.

In other embodiments, different from the above embodiments, the bottom wall of the aerosol-generating article 20 near the containment slot 37 is arranged with a second air-conducting portion 43 that is in communication with the second air inlet hole 41 to be inserted into the second air inlet hole 41, as well as for supporting the aerosol-generating article 20, such that the aerosol-generating article 20 is spaced apart from the bottom wall of the containment slot 37.

In some embodiments of the present disclosure, the housing 33 includes an outer shell 44 and an inner tube 45, which may be detachably connected or integrally molded between the outer shell 44 and the inner tube 45. The outer shell 44 defines the containment cavity 36, the atomizing assembly 10 is accommodated in the containment cavity 36, and the aerosol-generating article 20 is inserted into the inner tube 45. The inner tube 45 is disposed at an end of the outer shell 44 and defines the containment slot 37. An end of the inner tube 45 is inserted into the outer shell 44, and the other end of the inner tube 45 extends out of the outer shell 44 and forms a nozzle portion 46. The nozzle portion 46 has an outer periphery in the shape of a smooth arc. When the user performs a vaping action, the first aerosol and the second aerosol generated by the atomizing assembly 10 and the aerosol generating article 20 flow into the mouth of the user through the nozzle portion 46, respectively.

In some embodiments, when the heating method of the heating assembly 35 is electromagnetic heating, the coil 351, serving as the heating assembly 35, is disposed on an outer surface of the inner tube 45 and is electrically connected to the battery 32 through the control circuit board 31. The containment tube 21 in the aerosol-generating article 20 is made of a metal material or a metal material is arranged in the substrate section 22 in the aerosol-generating article 20, such as, copper, platinum, iron, etc. When the control circuit board 31 controls the battery 32 to supply power to the coil 351, the metal material cooperates with the coil 351 to heat and atomize the solid aerosol-generating substrate to generate the second aerosol.

In other embodiments, when the heating method of the heating assembly 35 is infrared heating, an inner surface of the inner tube 45 is coated with an infrared material or the inner tube 45 itself is made of an infrared material, and the infrared material emits infrared rays under an energized condition to heat and atomize the solid aerosol-generating substrate to generate the second aerosol.

In other embodiments, when the heating method of the heating assembly 35 is resistance heating, the heating assembly 35 may be a heating element arranged on the inner tube 45 and electrically connected to the battery 32 through the control circuit board 31. When the control circuit board 31 controls the battery 32 to supply power to the heating element, the heating element generates heat to atomize the solid aerosol-generating substrate to generate the second aerosol. Alternatively, the inner tube 45 itself is made of an electrically conductive material to serve as the heating assembly 35.

In some embodiments, the length of the aerosol-generating article 20 is slightly greater than the length of the inner tube 45. After the aerosol-generating article 20 is inserted into the inner tube 45, the filter section 24 of the aerosol-generating article 20 extends to the nozzle portion 46 and is slightly higher than the nozzle portion 46, which may satisfy the user's need to vape the first aerosol and the second aerosol at the same time, and also facilitate the disassembly of the aerosol-generating article 20 after the user has finished vaping.

In some embodiments, the outer shell 44 defines a first aperture (not shown), the first aperture being in communication with the liquid storage chamber 11 of the atomizing assembly 10. In this way, when there is insufficient liquid aerosol-generating substrate in the liquid storage chamber 11, the user can replenish the liquid aerosol-generating substrate into the atomizing assembly 10 through the first aperture. It is understood that under normal use of the atomizing assembly 10, the first aperture or the liquid storage chamber 11 is arranged with a sealing structure, such that the liquid in the liquid storage chamber 11 cannot leak through the first aperture.

In some embodiments, the outer shell 44 further defines a second aperture (not shown), and the atomizing assembly 10 can be assembled into the containment cavity 36 through the second aperture. In some embodiments, a bin cover (not shown) is arranged on the second aperture; the bin cover is slidably connected to the outer shell 44, or a side of the bin cover is connected to the outer shell 44 through a rotary shaft. By opening the bin cover, the atomizing assembly 10 can be assembled into or removed from the containment cavity 36 through the second aperture.

In the present disclosure, each of the electronic atomizing system 1 and the electronic atomizing device 30 includes a housing and an atomizing assembly, the housing defining a containment cavity a containment slot; a slot wall of the containment slot defines a first air inlet hole; the atomizing assembly is arranged in the containment cavity and configured to atomize the first aerosol-generating substrate to generate a first aerosol; an air outlet of the atomizing assembly is in communication with the first air inlet hole; the containment slot is configured to accommodate an aerosol-generating article and cooperate with the aerosol-generating article to form an air-conducting channel; an end of the air-conducting channel is in communication with the first air inlet hole, and the other end of the air-conducting channel is extended to a slot opening of the containment slot, such that the first aerosol generated by the atomizing assembly and a second aerosol generated by the aerosol-generating article flow through different airflow channels. Through the above arrangement, the first aerosol generated by the atomizing assembly 10 and the second aerosol generated by the aerosol-generating article 20 are respectively introduced into the mouth of the user through the slot opening, such that the aerosol generated in the atomizing assembly 10 may be effectively prevented from entering the aerosol-generating article 20 to be heated twice, thereby improving the user experience.

The above is only some embodiments of the present disclosure, and is not intended to limit the scope of the present disclosure. Any equivalent structure or equivalent process transformation utilizing the contents of the specification and the accompanying drawings of the present disclosure, or directly or indirectly utilized in other related technical fields, are all similarly included in the scope of the present disclosure.

## Claims

1. An electronic atomizing device, comprising:
a housing, defining a containment cavity and a containment slot; wherein a slot wall of the containment slot defines a first air inlet hole; and
an atomizing assembly, arranged in the containment cavity and configured to atomize a first aerosol-generating substrate for generating a first aerosol; wherein the first air inlet hole is configured to be in communication with an air outlet of the atomizing assembly;
wherein the containment slot is configured to accommodate an aerosol-generating article and cooperate with the aerosol-generating article to form an air-conducting channel; an end of the air-conducting channel is in communication with the first air inlet hole, and the other end of the air-conducting channel is extended to a slot opening of the containment slot, enabling the first aerosol generated by the atomizing assembly and a second aerosol generated by the aerosol-generating article to flow through different airflow channels.

2. The electronic atomizing device according to claim 1, wherein the slot wall of the containment slot further defines a second air inlet hole, the second air inlet hole being configured to communicate with an end of the aerosol-generating article near a bottom wall of the containment slot.

3. The electronic atomizing device according to claim 2, wherein the first air inlet hole and the second air inlet hole are both defined on the bottom wall of the containment slot.

4. The electronic atomizing device according to claim 3, wherein the atomizing assembly is disposed on the bottom wall of the containment slot, and an outer surface of the bottom wall of the containment slot is arranged with a first air-conducting portion that is in communication with the first air inlet hole; an end of the first air-conducting portion away from the first air inlet hole is connected to the air outlet of the atomizing assembly.

5. The electronic atomizing device according to claim 3, wherein an inner surface of the bottom wall of the containment slot is arranged with a second air-conducting portion that is in communication with the second air inlet hole; the second air-conducting portion is configured to abut against and be in communication with the end of the aerosol-generating article near the bottom wall of the containment slot, and to positionally limit the aerosol-generating article; the aerosol-generating article is spaced apart from the bottom wall of the containment slot.

6. The electronic atomizing device according to claim 5, wherein the second air inlet hole comprises a plurality of second air inlet holes, and the second air-conducting portion comprises a plurality of second air-conducting portions; the plurality of second air inlet holes and the plurality of second air-conducting portions are arranged in one-to-one correspondence; the plurality of second air inlet holes are disposed around the first air inlet hole.

7. The electronic atomizing device according to claim 1, wherein the housing comprises:
an outer shell, defining the containment cavity; and
an inner tube, disposed at an end of the outer shell and defining the containment slot;
wherein an end of the inner tube is inserted into the outer shell, and the other end of the inner tube extends out of the outer shell and forms a nozzle portion.

8. The electronic atomizing device according to claim 7, wherein,
the atomizing assembly comprises:
a liquid storage chamber, configured to store the first aerosol-generating substrate in a liquid state; and
an atomizing core, configured to atomize the first aerosol-generating substrate in the liquid state to generate the first aerosol;
the electronic atomizing device further comprises:
a heating assembly, disposed within the housing and configured to heat the aerosol-generating article to generate the second aerosol.

9. The electronic atomizing device according to claim 8, wherein the heating assembly is arranged on the inner tube, or the inner tube is the heating assembly.

10. The electronic atomizing device according to claim 8, further comprising:
a battery; and
a control circuit board, electrically connected to the battery, the atomizing core, and the heating assembly; wherein the control circuit board is configured to control the battery to supply power to the heating assembly and the atomizing core, respectively.

11. An electronic atomizing system, comprising:
the electronic atomizing device according to any one of claims 1-10; and
the aerosol-generating article, arranged in the containment slot, spaced apart from a side wall of containment slot, and configured to generate the second aerosol;
wherein the electronic atomizing device is configured to heat the aerosol-generating article.

12. The electronic atomizing system according to claim 11, wherein the aerosol-generating article is detachably arranged in the containment slot and comprises: a containment tube and a second aerosol-generating substrate disposed within the containment housing.

13. The electronic atomizing system according to claim 12, wherein the first aerosol-generating substrate is arranged inside a first end of the containment tube to form a substrate section, a filtering material is arranged inside a second end of the containment tube opposite to the first end to form a filter section, and a hollow section is formed in a middle portion of the containment tube; the substrate section is inserted into the containment slot, and the filter section extends out of the containment slot.

14. The electronic atomizing system according to claim 11, wherein the aerosol-generating article is spaced apart from a bottom wall of the containment slot, and an end of the aerosol-generating article near the containment slot is a sealed end.

15. The electronic atomizing system according to claim 11, wherein the electronic atomizing device is according to claim 5; the aerosol-generating article is spaced apart from a bottom wall of the containment slot; an end of the aerosol-generating article near the bottom wall of the containment slot defines a third air inlet hole; the second air inlet hole and the third air inlet hole are in communication with each other through the second air-conducting portion.

16. The electronic atomizing system according to claim 11, wherein the electronic atomizing device is according to claim 7; an end of the aerosol-generating article away from the bottom wall of the containment slot extends out of the nozzle portion.
